# EUROPEAN PATENT APPLICATION

(11) **EP 4 656 150 A1**
(43) Date of publication of application: **03.12.2025**
(21) Application number: 25179994.6
(22) Date of filing: 30.05.2025
(51) Int. Cl.: A61B 18/14, A61B 18/00, A61B 18/16

(54) **RETURN ELECTRODE CONFIGURATIONS FOR BIPOLAR SPHINCTEROTOMES**

(30) Priority: 30.05.2024 US 202463653609 P
(71) Applicant: Cook Medical Technologies, LLC, Bloomington, IN 47404 (US)
(72) Inventor: SIGMON Jr., John C., Winston-Salem, 27104 (US); PELHAM, Hunter, Winston-Salem, 27103 (US); ENTZ II, Michael W., Cary,, 27518 (US)
(74) Representative: Mathys & Squire

(57) **Abstract**

A bipolar sphincterotome includes: an elongate tubular member longitudinally extending from a proximal portion to a distal portion; a cutting wire longitudinally extending in the elongate tubular member, and extending to outside of the elongate tubular member via a cutting wire opening in the elongate tubular member; a return wire longitudinally extending from the proximal portion to the distal portion; and a return electrode electrically coupled to a return wire, where the return electrode includes a proximal cannula disposed proximal a cutting wire opening and a distal cannula disposed distal an anchor point, where both the proximal cannula and the distal cannula are electrically coupled to the return wire, and where both the proximal cannula and the distal cannula are metallic.

## Description

### TECHNICAL FIELD

The present invention relates generally to medical devices, and more particularly to sphincterotomes.

### BACKGROUND

A sphincterotome is a medical device that is used to perform a sphincterotomy, which involves cutting a sphincter muscle, such as the sphincter of Oddi. The sphincter muscle may need to be cut to relieve its constrictive nature and allow one or more medical devices through the muscle. For example, problems occurring in the biliary tree, such as the formation of bile duct stones or papillary stenosis, may be treated using medical devices that are delivered into the biliary tree. In order to access the biliary tree, the medical devices may pass through the sphincter of Oddi. To facilitate passage of the medical devices through the sphincter of Oddi, the sphincter muscle may be cut using a sphincterotome.

### BRIEF SUMMARY

The present describes various embodiments of a bipolar sphincterotome, and related electrosurgical systems, electrosurgical medical devices, methods of operation, and methods of assembly.

In one embodiment, a bipolar sphincterotome includes: an elongate tubular member longitudinally extending from a proximal portion to a distal portion; a cutting wire longitudinally extending in the elongate tubular member, and extending to outside of the elongate tubular member via a cutting wire opening in the elongate tubular member, wherein a distal end of the cutting wire is anchored to the elongate tubular member at an anchor point distal the cutting wire opening, and wherein a portion of the cutting wire outside of the elongate tubular member comprises a cutting edge; a return wire longitudinally extending from the proximal portion to the distal portion; and a return electrode electrically coupled to the return wire, the return electrode comprising a proximal cannula disposed proximal the cutting wire opening and a distal cannula disposed distal the anchor point, both the proximal cannula and the distal cannula electrically coupled to the return wire, and wherein the proximal cannula and the distal cannula are both metallic.

In another embodiment, a method of forming a return electrode on a distal portion of a bipolar sphincterotome includes: moving a proximal cannula to a first final position proximal a cutting wire opening of the bipolar sphincterotome and a distal cannula to a second final position distal an anchor point of the bipolar sphincterotome, wherein the proximal cannula and the distal cannula are both metallic; and electrically coupling a return wire of the bipolar sphincterotome to both the proximal cannula and the distal cannula.

Other embodiments are possible, and each of the embodiments can be used alone or together in combination. Accordingly, various embodiments are described below with reference to the accompanying drawings.

### BRIEF DESCRIPTION OF THE DRAWINGS

FIG. 1 shows a cross-sectional side view of an electrosurgical system including a bipolar sphincterotome electrically coupled to a power source.
FIG. 2 shows a perspective view of a distal portion of the bipolar sphincterotome of FIG. 1 in a curled position.
FIG. 3 shows cross-sectional axial view of the bipolar sphincterotome of FIG. 1 taken alone line 3-3.
FIG. 4 shows a perspective axial view of the bipolar sphincterotome of FIG. 1 from a distal end of the bipolar sphincterotome.
FIG. 5A shows a cross-sectional side view of a distal portion of the bipolar sphincterotome of FIG. 1, illustrating a first example configuration of a return electrode for the bipolar sphincterotome.
FIG. 5B shows a perspective side view of the distal portion of the bipolar sphincterotome of FIG. 1 with the first example configuration of the return electrode.
FIG. 5C shows a cross-sectional axial view of the sphincterotome of FIG. 1 with the first example configuration of the return electrode.
FIG. 6A shows a cross-sectional side view of the distal portion of the bipolar sphincterotome of FIG. 1, illustrating a second example configuration of a return electrode for the bipolar sphincterotome.
FIG. 6B shows a perspective side view of the distal portion of the bipolar sphincterotome of FIG. 1 with the second example configuration of the return electrode.
FIG. 7A shows a cross-sectional side view of the distal portion of the bipolar sphincterotome of FIG. 1, illustrating a third example configuration of a return electrode for the bipolar sphincterotome.
FIG. 7B shows a perspective side view of the distal portion of the bipolar sphincterotome of FIG. 1 with the third example configuration of the return electrode.
FIG. 7C shows a perspective top view of the distal portion of the bipolar sphincterotome of FIG. 1, with the third example configuration of the return electrode beginning to be coupled to an elongate tubular member from a distal end of the bipolar sphincterotome.
FIG. 7D shows another perspective top view of the distal portion of the bipolar sphincterotome of FIG. 1, with the third example configuration of the return electrode inserted over the elongate tubular member but not yet at its final position.
FIG. 7E shows another perspective top view of the distal portion of the bipolar sphincterotome of FIG. 1, with the third example configuration of the return electrode inserted over the elongate tubular member and at its final position.
FIG. 8A shows a cross-sectional side view of the distal portion of the bipolar sphincterotome of FIG. 1, illustrating a fourth example configuration of a return electrode for the bipolar sphincterotome.
FIG. 8B shows a perspective side view of the distal portion of the bipolar sphincterotome of FIG. 1 with the fourth example configuration of the return electrode.
FIG. 8C shows a perspective top view of the distal portion of the bipolar sphincterotome of FIG. 1, with the fourth example configuration of the return electrode beginning to be coupled to an elongate tubular member from a distal end of the bipolar sphincterotome.
FIG. 8D shows another perspective top view of the distal portion of the bipolar sphincterotome of FIG. 1, with the fourth example configuration of the return electrode inserted over the elongate tubular member but not yet at its final position.
FIG. 8E shows another perspective top view of the distal portion of the bipolar sphincterotome of FIG. 1, with the fourth example configuration of the return electrode inserted over the elongate tubular member and at its final position.
FIG. 9A shows a cross-sectional side view of the distal portion of the bipolar sphincterotome of FIG. 1, illustrating a fifth example configuration of a return electrode for the bipolar sphincterotome.
FIG. 9B shows a perspective side view of the distal portion of the bipolar sphincterotome of FIG. 1 with the fifth example configuration of the return electrode.
FIG. 9C shows a perspective axial view of the distal portion of the bipolar sphincterotome of FIG. 1 from the distal end with the fifth example configuration of the return electrode.
FIG. 10A shows a cross-sectional side view of the distal portion of the bipolar sphincterotome of FIG. 1, illustrating a sixth example configuration of a return electrode for the bipolar sphincterotome.
FIG. 10B shows a perspective side view of the distal portion of the bipolar sphincterotome of FIG. 1 with the sixth example configuration of the return electrode.
FIG. 10C shows a perspective bottom view of the distal portion of the bipolar sphincterotome of FIG. 1, with the sixth example configuration of the return electrode.
FIG. 11A shows a cross-sectional side view of the distal portion of the bipolar sphincterotome of FIG. 1, illustrating a seventh example configuration of a return electrode for the bipolar sphincterotome.
FIG. 11B shows a perspective side view of the distal portion of the bipolar sphincterotome of FIG. 1 with the seventh example configuration of the return electrode.
FIG. 11C shows a perspective bottom view of the distal portion of the bipolar sphincterotome of FIG. 1, with the seventh example configuration of the return electrode.

### DETAILED DESCRIPTION

The present description describes various embodiments of a sphincterotome, electrosurgical systems, electrosurgical medical devices, and related methods having a metallic return electrode.

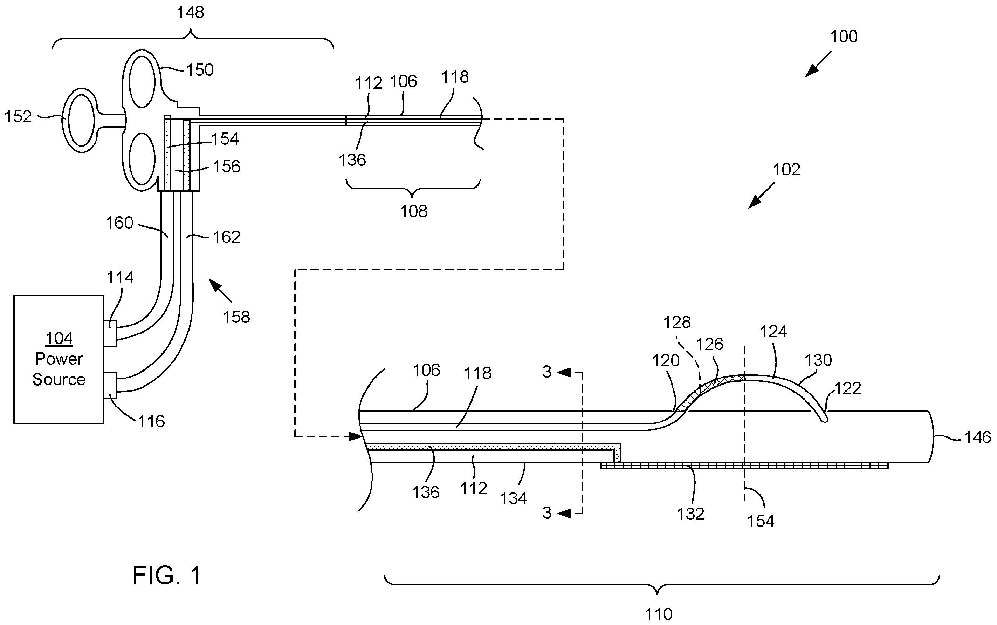

Fig. 1 shows a partial cross-sectional side view of an example electrosurgical system 100 that includes a bipolar sphincterotome 102 coupled to a power source 104. Other embodiments of the present description may include only the bipolar sphincterotome 102 without the power source 104.

The bipolar sphincterotome 102 may include an elongate tubular member 106 (e.g., a catheter) that longitudinally extends from a proximal portion 108 to a distal portion 110. The elongate tubular member 106 may include a body 112 extending from the proximal portion 108 to the distal portion 110. The body 112 may be made of various suitable materials, including non-conductive materials, such as polytetrafluoroethylene (PTFE), perfluoroalkoxy (PFA), polyethylene, nylon, or fluorinated ethylene, as non-limiting examples.

The bipolar sphincterotome 102 may further include an active path and a return path. In general, the active path and the return path are each conductive elements, or each a network or assembly of elements, that are configured to deliver electrical current between the power source 104 and a treatment site (e.g., a sphincter muscle) within a patient. As shown in Fig. 1, the power source 104 (e.g., an electrosurgical unit (ESU) or a radio frequency (RF) generator) may be configured to output electrical current via a pair of ports, including an active port 114 and a return port 116. The active path may be configured to electrically couple to the active port 114, and the return path may be configured to electrically couple to the return port 116.

In addition, at a treatment site within a patient, a distal portion of the active path is configured to contact a portion of tissue at the treatment site, and a distal portion of the return path is configured to contact another portion of the tissue. In further detail, for the bipolar configuration such as in Fig. 1, when the distal portions of the active and return paths are contacting the tissue, the power source, the active and return paths, and the tissue at the treatment site form an electrical circuit through which electrical current generated by the power source 104 may flow. The electrical current flowing through the tissue may produce a certain effect on the tissue, such as cutting, ablation, or coagulation, as non-limiting examples. In addition, for an electrosurgical procedure such as a sphincterotomy, the electrical current may be an alternating current (AC), such as a radio frequency (RF) current. Accordingly, when the electrical circuit is formed, depending on its polarity, electrical current generated by the power source 104 may flow from the active port 114 of the power source 104, through the active path of the bipolar sphincterotome 102, through the tissue at the treatment site, and then through the return path back to the return port 116; or may flow from the return port 116, through the return path, through the tissue at the treatment site, and then through the active path back to the active port 114.

Additionally, the active path of the sphincterotome 102 may include a conductive cutting wire or other elongate conductive element 118 (also called herein an active wire) that longitudinally extends from the proximal portion 108 to the distal portion 110. In various embodiments, such as shown in Fig. 1, the cutting wire 118 may longitudinally extend within the body 112 except at the distal portion 110, where the cutting wire 118 exits at an exit port or opening 120 (also called herein a cutting wire exit port, a cutting wire opening, an active wire exit port, or an active wire opening) from within the body 112 to outside of the body 112, and distally extends outside of the body 112 from the exit port 120 to an anchor point 122, where a distal end of the cutting wire 118 is secured or affixed to the body 112. The portion of the cutting wire 118 that is outside of the body 112 may be referred to as a cutting edge 124, and is an electrode part of the cutting wire 118 that is configured to contact tissue at the treatment site. For example, during a sphincterotomy, the cutting edge 124 is a part of the cutting wire 118 that is configured to contact a sphincter muscle and cut the sphincter muscle when electrical current is delivered to it.

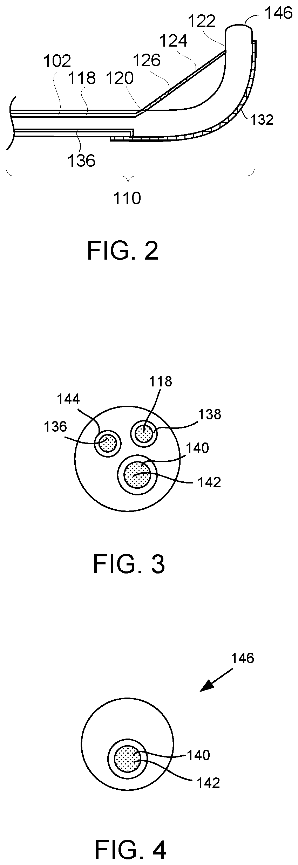

In addition, the cutting wire 118 may be longitudinally movable within the body 112, such as by being disposed within a cutting wire lumen (not shown in Fig. 1) that longitudinally extends from the proximal portion 108 to the distal portion 110. By being longitudinally movable within the body 112, the cutting wire 118 may be configured to move the distal portion 110 of the elongate tubular member 106 between curled (or bowed) and uncurled positions. Fig. 1 shows the distal portion 110 in an uncurled position. Fig. 2 shows the distal portion 110 in a curled position. For example, when the cutting wire 118 is proximally pulled relative to the elongate tubular member 106, the distal end of the cutting wire 118 may exert a force on the elongate tubular member 106 at the anchor point 122 that causes the distal portion 110 to curl in a curling direction, as shown in Fig. 2. When the distal portion 110 is in a curled position due to the cutting wire 118 being proximally pulled, the cutting edge 124 may be relatively taut and in a cutting position. An operator operating the bipolar sphincterotome 102 to perform a sphincterotomy may operate the sphincterotome 102 to proximally pull the cutting wire 118 in order to configure the distal portion 110 in the curled position and the cutting edge 124 to be taut in the cutting position in order to cut the tissue.

In addition, as shown in Figs. 1 and 2, the sphincterotome 102 may include a cover 126 that covers and/or is disposed over a portion of the cutting edge 124. As used herein the portion of the cutting edge 124 that is covered by the cover 126 is referred to as an insulated portion 128, and the portion of the cutting edge 124 that is not covered by the cover 126 is referred to an exposed portion 130.

The cover 126 may prevent the portion of the cutting edge 124 it is covering from contacting tissue and delivering electrical current to the tissue. The cover 126 may serve as and also be referred to as a protector, in that it insulates and/or protects tissue from coming into contact with and being cut by the portion of the cutting edge 124 being covered by the cover 126. Accordingly, by having a portion covered by the cover 126, the entirety of the cutting edge 126 (e.g., the portion of the cutting wire 118 disposed outside of the elongate tubular member 106) is not configured to contact and cut tissue. The cover 126 may reduce the surface area of the cutting edge 124 configured or able to contact tissue, compared to if no cover was present. This, in turn, may improve the cutting ability of the cutting edge 124, and the overall ability of the sphincterotome 102 to perform a sphincterotomy. In particular, the cover 126 may improve or enhance performance of the sphincterotome 102 by: concentrating the current density to a smaller portion of the cutting edge 124 able to contact tissue, by preventing the proximal portion of the cutting edge 124 from cutting tissue that is not desired to be cut, and/or by preventing undesirable sparking.

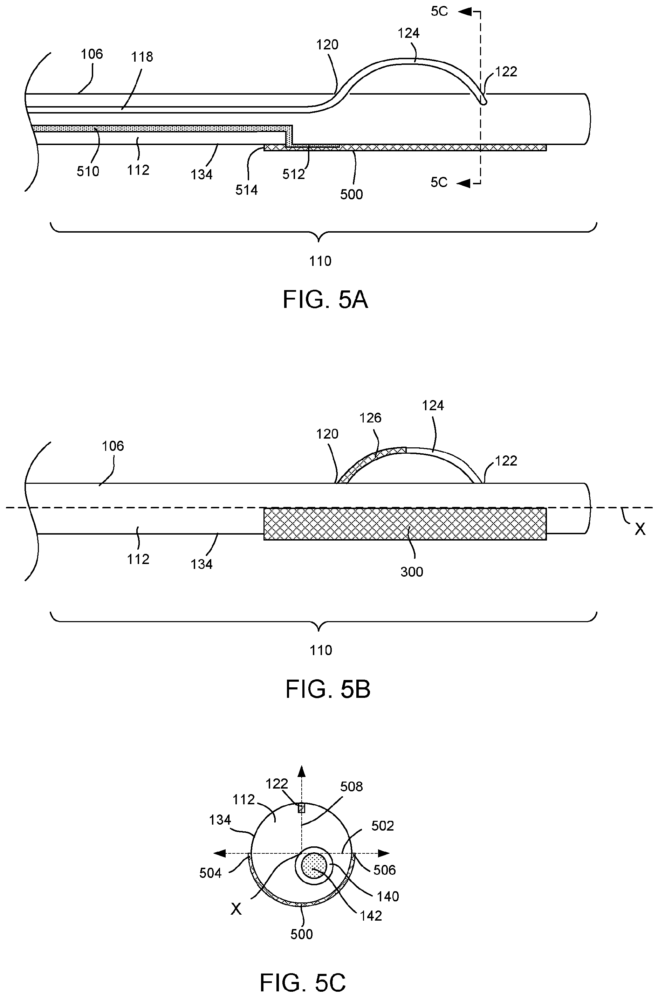

As described, the bipolar sphincterotome 102 shown in Figs. 1 and 2, having a bipolar configuration, has its return path integrated with, or a part of, the elongate tubular member 106. For example, the return path of the bipolar sphincterotome 102 may include a return electrode 132 at the distal portion 110. In general, the return electrode 132 is a conductive component of the return path that is exposed from within the elongate tubular member 106 and is configured to contact tissue at the treatment site. Additionally, in various embodiments, such as shown in Fig. 1, the return electrode 132 may cover, be disposed over or about, and/or affixed or adhered to an outer surface 134 of the body 112 of the elongate tubular member 106. Further details of various configurations of the return electrode 132 are described below with reference to Figs. 5A-11C.

In addition to the return electrode 132, the return path may also include a return wire or other elongate conductive element 136 that longitudinally extends from the proximal portion 108 to the distal portion 110 and that connects to the return electrode 132. In general, the return wire 136 longitudinally extends alongside the cutting wire 118 from the proximal portion 108 to the distal portion 110. In some implementations, such as shown in Fig. 1, the return wire 130 may longitudinally extend within the body 112, such as by extending within a return lumen (not separately shown from the return wire 136 in Figs. 1 and 2) of the elongate tubular member 106. As shown in Fig. 1, at the distal portion 110, the return wire 136 (or another conductive element of the return path) may extend from within the body 112 to the outer surface 134 to connect to the return electrode 136. In other implementations, at least a portion of the return wire 136 extends outside of, such as alongside, the body 112 from the proximal portion 108 to the distal portion 110. Various ways of connecting the return wire 136 to the return electrode 132 and/or having the return path longitudinally extend from the proximal portion 108 to the distal portion 110 may be possible.

Fig. 3 shows an axial cross-sectional view of the bipolar sphincterotome of Figs. 1 and 2, taken along line 3-3. The axial cross-section in Fig. 3 is generally representative of an example axial cross-section the bipolar sphincterotome 102 proximal the opening 120 and proximal the proximal end of the return electrode 126. As shown in Fig. 3, the bipolar sphincterotome 100, proximal the opening 120 and/or the proximal end of the return electrode 126, may include a plurality of lumens longitudinally extending within the body 112 from the proximal portion 108 to the distal portion 110. The plurality of lumens may include a cutting wire lumen (also called herein an active wire lumen) 138 in which the cutting wire 118 is movably disposed. In addition, the plurality of lumens may a wireguide lumen 140 in which an elongate wireguide 142 is movably disposed. In operation, the bipolar sphincterotome 102 may be delivered to a treatment site within a patient by inserting the wireguide lumen 140 over the wireguide 142 and distally moving the bipolar sphincterotome 102 over the wireguide 142 from outside of the patient to the treatment site within the patient.

In addition, the plurality of lumens may include an additional lumen 144 used for one or more functions. In some implementations, the additional lumen 144 is used an injection lumen in which fluid is injected for delivery to the treatment site. In addition or alternatively, in some implementations, the additional lumen 144 has disposed therein the return wire 136. In this way, the additional lumen has two functions-to deliver fluid through the elongate tubular member 106 to the treatment site and to house the return wire 136. In such implementations, fluid may flow around the return wire in order to be delivered to the treatment site. In other implementations, the plurality of lumens may include four lumens, including two additional lumens, where a first additional lumen is used for fluid delivery and a second additional lumen houses the return wire 136. In still other implementations, the plurality of lumens may include one additional lumen as shown in Fig. 3, but the return wire 136 may be disposed outside of the one additional lumen and otherwise not disposed within any of the plurality of lumens, such as by being embedded within the body 112 of the elongate tubular member 106. In other implementations, the return wire 136 is disposed outside of and longitudinally extends alongside the body 112 of the elongate tubular member 106, as such is not disposed in any of the plurality of lumens longitudinally extending through the body 112.

Fig. 4 is an axial view of a distal end 146 of the bipolar sphincterotome 102. For simplicity, the cutting edge 124 and the return electrode 132 are not shown in the view of Fig. 4. In some implementations such as shown in Fig. 4, only one of the plurality of lumens-i.e., the wireguide lumen 140-longitudinally extends all the way to the distal end 146. As indicated in Fig. 1, the cutting wire lumen 138 extends to the opening 120, and so does not longitudinally extend all way to the distal end 146. Additionally, in some implementations as shown in Fig. 4, the additional lumen 144 may merge with the wireguide lumen 140 proximal the distal end 146, such that the wireguide lumen 140 (or the resulting merged lumen) housing the wireguide 142 is the only lumen to longitudinally extend to the distal end 146. In such implementations, the additional lumen 144 may not need to extend all the way to the distal end 146 for purposes of the return wire 136 since the return wire 136 terminates with the return electrode 132 before the distal end 146. Additionally, for implementations where the additional lumen is used to deliver fluid, the fluid then exits the elongate tubular member 106 at the distal end 146 via the wireguide lumen 140 (or the resulting merged lumen). A configuration that reduces the number of lumens extending to the distal end 146 such as shown in Fig. 4 may desirably allow for a distal-most part of the distal portion 110 (e.g., a distal tip) to a have a reduced or smaller outer diameter compared to the outer diameter of the rest of the elongate tubular member 106 without losing functionality. Other implementations may have one or more lumens, in addition to the wireguide lumen 140, that extend all the way to the distal end 146. For example, other implementations may configure the additional lumen 144 to longitudinally extend all the way to the distal end 146 separate from and/or independent of the wireguide lumen 140.

Additionally, referring to Figs. 3 and 4, the relative axial positioning of the plurality of lumens, including the cutting wire lumen 138, the wireguide lumen 140, and additional lumen 144, is illustrated as merely one example. Different axial positions of one or more of the plurality of lumens in any of various other implementations is possible.

Referring to Fig. 1, the sphincterotome 102 may also include a handle assembly 148 operably coupled to the elongate tubular member 106 and the cutting wire 118. In various embodiments, the handle assembly 148 may include a plurality of portions that are movable relative to each other to control longitudinal movement of the cutting wire 118 relative to the elongate tubular member 106. For example, in the example configuration shown in Fig. 1, the handle assembly 148 may include a first portion 150 in the form of finger rings and a second portion 152 in the form of a thumb ring through which an operator may insert his/her fingers and thumb, respectively, when grasping the handle assembly 148. The finger rings 150 may be operatively coupled to the cutting wire 118, such that movement of the finger rings 150 relative to the rest of the handle assembly 148 may cause the cutting wire 118 to longitudinally move relative to the elongate tubular member 106. Correspondingly, upon grasping the handle assembly 148, the operator may open and close his/her hand to move the finger rings 150 farther away and closer to the thumb ring 152, respectively. Moving the finger rings 150 toward the thumb ring 152 may proximally pull the cutting wire 118, which in turn may move the distal portion 110 into the curled position, such as shown in Fig. 2. Additionally, moving the finger rings 150 away from the thumb ring 152 may move the distal portion 110 into the uncurled position, such as shown in Fig. 1. The handle assembly 148 shown in Fig. 1 is merely one example, and various other configurations of a handle assembly to facilitate longitudinal movement of the cutting wire 118 relative to the elongate tubular member 106 may be possible.

Also, in various implementations such as shown in Fig. 1, the handle assembly 148 may include components of the active path. For example, a proximal portion of the cutting wire 118 may extend in the handle assembly 148. Additionally, as shown in Fig. 1, the handle assembly 148 may include a conductive active element (or an assembly of active elements in various implementations) 154 that connects to the cutting wire 118, and that is used to electrically couple the cutting wire 118 to the active port 114 of the power source 104. The handle assembly 148 may similarly include a conductive return element (or an assembly of return elements in various implementations) 156 that connects to the return wire 136, and that is used to electrically couple the return wire 136 to the return port 116 of the power source 104.

Additionally, in various implementations, the electrosurgical system 100 may include electrical cabling 158 configured to electrically couple the power source 104 to the bipolar sphincterotome 102. The electrical cabling 158 may include an active cable or wire 160 configured to connect the active port 114 to the active path of the handle assembly 148 and/or the bipolar sphincterotome 102. For example, the active cable 160 may connect to the active element 154 of the handle assembly 148. Similarly, the electrical cabling 158 may include a return cable or wire 162 configured to connect the return port 116 to the return path of the handle assembly 148 and/or the bipolar sphincterotome 102. For example, the return cable 162 may connect to the return element 156 of the handle assembly 148. In various embodiments, the electrical cabling 158 may be considered a component of the bipolar sphincterotome 102. In other embodiments, the electrical cabling 158 may be considered a component of the electrosurgical system 100 that is separate from the bipolar sphincterotome 102. In various implementations, the active cable 160 and/or the return cable 162 may be fixedly connected or attached to the handle assembly 148. In other implementations, the active cable 160 and/or the return cable 162 may be removably connected or attached to the handle assembly 148.

The following with reference to Figs. 5A-11C describes various structural configurations for the return electrode 132. For at least some of these structural configurations, the return electrode may be metallic-i.e., the return electrode 132 is at least partially made of one or more pieces of metal. However, other structural configurations may be similarly shaped but made of different materials, such as conductive ink, paste, foil, wires, or a shrink tube that includes conductive material and/or conductive inclusions, or any of various combinations thereof, as non-limiting examples.

Figs. 5A-5C show an example implementation of the distal portion 110 of the bipolar sphincterotome 102 with a return electrode 500, a configuration of which may be representative of a first example configuration of the return electrode 132 of Figs. 1 and 2. Fig. 5A shows a cross-sectional side view of the distal portion 110 with the return electrode 500. Fig. 5B shows a perspective side view of the distal portion 110 with the return electrode 500. Fig. 5C shows an axial cross-sectional view of the distal portion 110 with the return electrode 500, taken along line 5C-5C.

In at least some implementations, the return electrode 500 may include and/or may be made of a single and/or a continuous piece of conductive material. In some implementations, the return electrode 500 includes and/or is made of a single and/or continuous piece of metal, such as sheet metal for example. In addition or alternatively, the return electrode 500 may be coupled and/or affixed to the outer surface 134 of the elongate tubular member 106 in any of various ways, such as by being glued or bonded (e.g., using an epoxy or other adhesive), crimped, and/or press-fit to and/or about the outer surface 134 of the elongate tubular member 106, as non-limiting examples. In addition or alternatively, the return electrode 500 may be fixedly attached to the body 112 of the elongate tubular member 106 by having at least a portion that grasps or grips the body 112, such as by being embedded in and/or by penetrating into the body 112. For example, a proximal end, a distal end, and/or one or both of the circumferential edges of the return electrode 500 may be folded or curled into the body 112 to form a grasping or gripping engagement with the body 112. In addition or alternatively, an inner surface of the return electrode 500 may include one or more spikes, pins, or other similar sharp or piercing structure that can penetrate and grip onto the body 112 in order for the return electrode 500 to be affixed, and/or enhance a coupling or attachment that the return electrode 300 has, to the body 112 of the elongate tubular member 106.

In addition, in some implementations as shown in Figs. 5B and 5C, the return electrode 500 may circumferentially extend about halfway around the outer surface 134 of the body 112 of the elongate tubular member 106-i.e., a circumferential length of the return electrode 500 is about half (e.g., within plus-or-minus 5%) of the circumference of the elongate tubular member 106. In other implementations, the return electrode 500 may circumferentially extend more than about halfway or less than about halfway around the outer surface 134 of the body 112 of the elongate tubular member 106. In addition, in any of various implementations, the circumferential length of the return electrode 500 may be constant or may vary over the longitudinal length of the return electrode 500. For example, at any two axial cross-sections over the longitudinal length of the return electrode 500, the circumferential lengths of the return electrode 500 may be the same as or different from each other.

In addition, at any given axial cross-section over a longitudinal length of the return electrode 500, the return electrode 500 may have a circumferential orientation about the outer surface 134 of the body 112 of the elongate tubular member 106. Suppose a geometric plane in which a longitudinal axis X of the elongate tubular member 106 extends, where such geometric plane also intersects the opening 120 and/or the anchor point 122. Over a given axial cross-section, the circumferential orientation may be defined by a geometric line extending through or intersecting circumferential ends or edges of the return electrode 500 and the geometric plane.

For example, in some implementations as shown in Figs. 5B and 5C, over an axial cross-section including the anchor point 122, the return electrode 500 may be circumferentially disposed about the outer surface 134 of the body 112 in that a geometric line 502 intersecting opposing circumferential ends or edges 504, 506 of the return electrode 500 may be about perpendicular (e.g., within plus-or-minus 5% of 90 degrees) to the geometric plane, denoted by a geometric line 508 intersecting an origin or center of the cross-section (e.g., the longitudinal axis) and the anchor point 122. In other implementations, such a geometric line 502 intersecting opposing circumferential ends 504, 506 may not be about perpendicular to the geometric line 508 intersecting the origin and the anchor point. Similarly, over a given axial cross-section over a longitudinal length of the return electrode 500, the circumferential orientation of the return electrode 500 may be such that a geometric line extending between circumferential ends of the return electrode 500 may be extend about perpendicular to the plane, or may not be about perpendicular to the plane. In addition or alternatively, circumferential orientations of the return electrode 500 at any two axial cross-sections over the longitudinal length of the return electrode 500 may be the same as or different than each other.

Additionally, as shown in Fig. 5A, in some implementations, a return wire 510, which may be representative of an example configuration of the return wire 136 of Figs. 1-3, may longitudinally extend from within to outside of the elongate tubular member 106 at the distal portion 110 to electrically connect to the return electrode 500. In addition, the return wire 510 may form the electrical connection with the return electrode in any of various ways. For example, in some implementations, the return wire 510 may directly physically connect or attach to the return electrode 500. In other implementations, the return wire 510 may be indirectly physically connected or attached to the return electrode 500, in that one or more conductive elements, such as solder, conductive ink, or any of various other types of conductive components may be used to physically and electrically coupled the return wire 510 and the return electrode 500.

In addition, in some implementations such as shown in Fig. 5A, a distal portion 512 of the return electrode 510 that is outside of the elongate tubular member 106 may be disposed between the outer surface 134 of the body 112 and the return electrode 500. In this context, the distal portion 512 may be considered to be disposed underneath and/or covered by the return electrode 500, which may ensure physical and electrical connection between the return electrode 500 and the return wire 510. Other implementations are possible however. For example, in other implementations, the return wire 510 may extend through the return electrode 500, such as through a hole or aperture in the return electrode 500, and the distal portion 512 is disposed on and physically and electrically connected to an outer surface of the return electrode 500. In this way, the return electrode 500 is disposed between the outer surface 134 of the body 112 of the elongate tubular member 106 and the return electrode 500. Various ways of configuring the return wire 510 to extend from within to outside of the elongate tubular member 106 to electrically connect to the return electrode 500 may be possible.

Additionally, in some implementations as shown in Figs. 5A and 5B, the return electrode 500 may proximally extend past the opening 120 and proximally where the return wire 510 extends from within to outside of the elongate tubular member 106. That is, a proximal end 514 of the return electrode 500 may be positioned proximal the opening 120 and proximal to where the return wire 510 extends from within to outside of the elongate tubular member 106. In other embodiments, the proximal end 514 of the return electrode 500 may be longitudinally even with, or distal to, where the return wire 510 extends from within the outside of the elongate tubular member 106. Various ways of configuring the proximal end 514 of the return electrode 500 relative to where the return wire 514 exits from within the elongate tubular member 106 are possible.

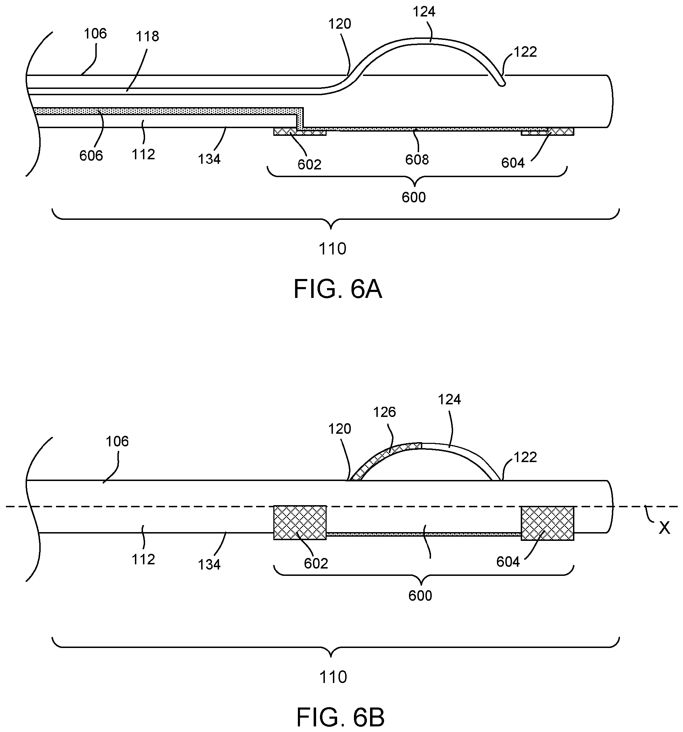

Figs. 6A and 6B show an example implementation of the distal portion 110 of the bipolar sphincterotome 102 with a return electrode 600, a configuration of which may be representative of a second example configuration of the return electrode 132 of Figs. 1 and 2. As shown in Figs. 6A and 6B, the return electrode 600 may include a plurality of metallic semi-rings or semi-ring structures, including a proximal semi-ring 602 and a distal semi-ring 604. In general, the proximal semi-ring 602 is disposed proximal the distal semi-ring 604. Otherwise stated, the distal semi-ring is disposed distal the proximal semi-ring 602. Also, as used herein, a semi-ring is a semi-circular or arc-shaped structure that circumferentially extends halfway or less than halfway around an outer surface of an elongate tubular member. In the implementation shown in Figs. 6A, 6B, the proximal and distal semi-rings 602, 604 each circumferentially extend halfway around the elongate tubular member 106. In other implementations, one or both of the proximal and distal semi-rings 602, 604 circumferentially extend less than halfway around. In addition or alternatively, in any of various implementations, the proximal and distal semi-rings 602, 604 may have circumferential lengths that are the same as or different from each other. In addition or alternatively, in any of various implementations, the proximal and distal semi-rings 602, 604 may have respective circumferential orientations that are the same as or different from each other.

Additionally, as shown in Fig. 6A, in some implementations, a return wire 606, which may be representative of an example configuration of the return wire 136 of Figs. 1-3, may longitudinally extend from within to outside of the elongate tubular member 106 at the distal portion 110 to electrically connect to the return electrode 600. In particular of these implementations such as in Fig. 6A, a distal portion 608 of the return wire 606 disposed outside of the elongate tubular member 106 may longitudinally extend and connect to the distal semi-ring 604 such that the return wire 606 is electrically connected to both the proximal and distal semi-rings 602, 604, and such that the proximal and distal semi-rings 602, 604 are electrically connected to each other and/or both the proximal and distal semi-rings 602, 604 are both part of the same return path. In this way, the distal portion 608 is disposed between the outer surface 134 of the elongate tubular member 106 and the proximal semi-ring 602 and between the outer surface 134 of the elongate tubular member 106 and the distal semi-ring 604. Various other ways of physically and/or electrically connecting the return wire 606 to the proximal and distal semi-rings 602, 604, such as through using one or more of any of various types of conductive components or elements, are possible.

Additionally, similar to the return electrode 500, the proximal and distal semi-rings 602, 604 may each include and/or may be made of a single and/or a continuous piece of conductive material, such as sheet metal or other type of metal. Accordingly, in the implementations shown in Figs. 6A and 6B, the metallic materials making up the proximal and distal semi-rings 602, 604 are longitudinally physically separated from each other.

In addition or alternatively, the proximal and distal semi-rings 602, 604 may be coupled and/or affixed to the outer surface 134 of the elongate tubular member 106 in any of various ways, such as by being glued or bonded (e.g., using an epoxy or other adhesive), crimped, and/or press-fit to and/or about the outer surface 134 of the elongate tubular member 106, as non-limiting examples. In addition or alternatively, in some implementations, one or both of the proximal and distal semi-rings 602, 604 may be fixedly attached to the body 112 of the elongate tubular member 106 by having at least a portion that grasps or grips the body 112, such as by being embedded in and/or by penetrating into the body 112. For example, proximal and/or distal ends of one or both of the proximal semi-ring 602 and/or the distal semi-ring 604 may be folded or curled into the body 112 to form a grasping or gripping engagement with the body 112. In addition or alternatively, an inner surface of one or both of the proximal and distal semi-rings 602, 604 may include one or more spikes, pins, or other similar sharp or piercing structure that can penetrate and grip onto the body 112 in order for the proximal semi-ring 602 and/or the distal semi-ring 604 to be affixed, and/or to enhance a coupling or attachment that the proximal semi-ring 602 and/or the distal semi-ring 604 has, to the body 112 of the elongate tubular member 106.

Also, in any of various implementations, the proximal end and the distal end of the proximal semi-ring 602 are longitudinally disposed relative to the opening 120 in any of various ways. For example, in the implementation shown in Figs. 6A, 6B, both a proximal end and a distal end of the proximal semi-ring 602 are longitudinally disposed proximal the opening 120. In other implementations, both the proximal end and the distal end of the proximal semi-ring 602 are longitudinally disposed distal the opening 120. In other implementations, the proximal end is disposed proximal the opening 120 and the distal end is disposed distal the opening 120. In still other implementations, the proximal end is proximal the opening 120 and the distal end is longitudinally aligned or even with the opening 120; or the proximal end is longitudinally aligned or even with the opening 120 and the distal end is distal the opening 120. Various ways of positioning the proximal and distal ends of the proximal semi-ring 602 relative to the opening 120 are possible.

Similarly, in any of various implementations, the proximal end and the distal end of the distal semi-ring 604 are longitudinally disposed relative to the anchor point 122 in any of various ways. For example, in the implementation shown in Figs. 6A, 6B, both a proximal end and a distal end of the distal semi-ring 604 are longitudinally disposed distal the anchor point 122. In other implementations, both the proximal end and the distal end of the distal semi-ring 604 are longitudinally disposed proximal the anchor point 122. In other implementations, the proximal end is disposed proximal the anchor point 122 and the distal end is disposed distal the anchor point 122. In still other implementations, the proximal end is proximal the anchor point 122 and the distal end is longitudinally aligned or even with the anchor point 122; or the proximal end is longitudinally aligned or even with the anchor point 122 and the distal end is distal the anchor point 122. Various ways of positioning the proximal and distal ends of the distal semi-ring 604 relative to the anchor point 122 are possible.

Also, in the implementation shown in Figs. 6A, 6B, the return electrode 600 includes two metallic semi-rings 602, 604. Other implementations may include more than two metallic semi-rings (e.g., three, four, five, etc.) longitudinally spaced apart from each other and electrically connected to each other. In at least some of these implementations, the proximal semi-ring 602 may be a proximal-most semi-ring of the plurality of semi-rings, and/or the distal semi-ring 604 may be a distal-most semi-ring of the plurality of semi-rings. In addition or alternatively, at least one of the semi-rings may be entirely disposed distal the opening 120 and proximal the anchor point 122 and/or entirely longitudinally extend alongside the cutting edge 124. Various way of configuring three or more semi-rings relative to the opening 120, the anchor point 122, and/or the cutting edge 124 are possible.

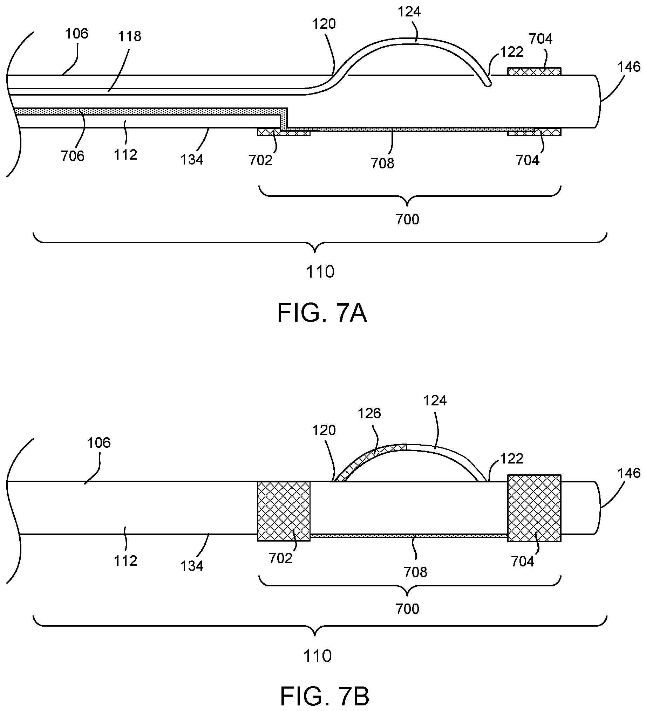

Figs. 7A-7E show an example implementation of the distal portion 110 of the bipolar sphincterotome 102 with a return electrode 700, a configuration of which may be representative of a third example configuration of the return electrode 132 of Figs. 1 and 2. The return electrode 700 may be similar to the configuration of the return electrode 600 in Figs. 6A, 6B, except instead of including semi-rings, the return electrode 700 may include a plurality of cannulas, including a proximal cannula 702 and a distal cannula 704. In general, the proximal cannula 702 is disposed proximal the distal cannula 704. Otherwise stated, the distal cannula 704 is disposed distal the proximal cannula 702. Also, as used herein, a cannula is similar to a semi-ring in that it is a generally round and/or ring-like structure that can circumferentially extend over or about at least a portion of the outer surface 134 of the elongate tubular member 106. However, in contrast to a semi-ring, a cannula, as used herein, circumferentially extends more than halfway around the outer surface 134 of the elongate tubular member 106. A given cannula may be circumferentially continuous in that it circumferentially extends completely around the outer surface 134. Alternatively, a given cannula may be circumferentially discontinuous in that it does not circumferentially extend completely around the outer surface 134. Unlike a cannula that is circumferentially continuous, a cannula that is circumferentially discontinuous may have opposing circumferential ends that are disconnected or separated from each other.

In the example implementation in Figs. 7A-7E, the proximal cannula 702 is circumferentially discontinuous and the distal cannula 704 is circumferentially continuous. In other implementations, the proximal and distal cannulas 702, 704 are both circumferentially discontinuous or are both circumferentially continuous. In any of various implementations where the proximal and distal cannulas 702, 704 are both circumferentially discontinuous, the proximal and distal cannulas have circumferential lengths that are the same as or different from each other.

Additionally, as shown in Fig. 7A, a return wire 706, which may be representative of an example configuration of the return wire 136 of Figs. 1-3, may be configured similarly as the return wire 606 of Figs. 6A, 6B. That is, the return wire 706 may longitudinally extend from within to outside of the elongate tubular member 106 at the distal portion 110 to electrically connect to the return electrode 700. Similar to the return wire 606, the return wire 706 may include a distal portion 708 disposed outside of the elongate tubular member 106 that longitudinally extends and connects to the distal cannula 704 such that the return wire 706 is electrically connected to both the proximal and distal cannulas 702, 704, and such that the proximal and distal cannulas 702, 704 are electrically connected to each other and/or both the proximal and distal cannulas 702, 704 are both part of the same return path. In this way, the distal portion 708 is disposed between the outer surface 134 of the elongate tubular member 106 and the proximal cannula 702 and between the outer surface 134 of the elongate tubular member 106 and the distal cannula 704. Various other ways of physically and/or electrically connecting the return wire 706 to the proximal and distal cannulas 702, 704, such as through using one or more of any of various types of conductive components or elements, are possible.

Additionally, similar to the proximal and distal semi-rings 602, 604, the proximal and distal cannulas 702, 704 may each include and/or may be made of a single and/or a continuous piece of conductive material, such as sheet metal or other type of metal. Accordingly, in the implementations shown in Figs. 7A-7D, the metallic materials making up the proximal and distal cannulas 702, 704 are longitudinally physically separated from each other.

In addition or alternatively, the proximal and distal cannulas 702, 704 may be coupled and/or affixed to the outer surface 134 of the elongate tubular member 106 in any of various ways, such as by being glued or bonded (e.g., using an epoxy or other adhesive), crimped, and/or press-fit to and/or about the outer surface 134 of the elongate tubular member 106, as non-limiting examples. In addition or alternatively, in some implementations, one or both of the proximal and distal cannulas 702, 704 may be fixedly attached to the body 112 of the elongate tubular member 106 by having at least a portion that grasps or grips the body 112, such as by being embedded in and/or by penetrating into the body 112. For example, proximal and/or distal ends of one or both of the proximal cannula 702 and/or the distal cannula 704 may be folded or curled into the body 112 to form a grasping or gripping engagement with the body 112. In addition or alternatively, an inner surface of one or both of the proximal and distal semi-rings 702, 704 may include one or more spikes, pins, or other similar sharp or piercing structure that can penetrate and grip onto the body 112 in order for the proximal cannula 702 and/or the distal cannula 704 to be affixed, and/or to enhance a coupling or attachment that the proximal cannula 702 and/or the distal cannula 704 has, to the body 112 of the elongate tubular member 106.

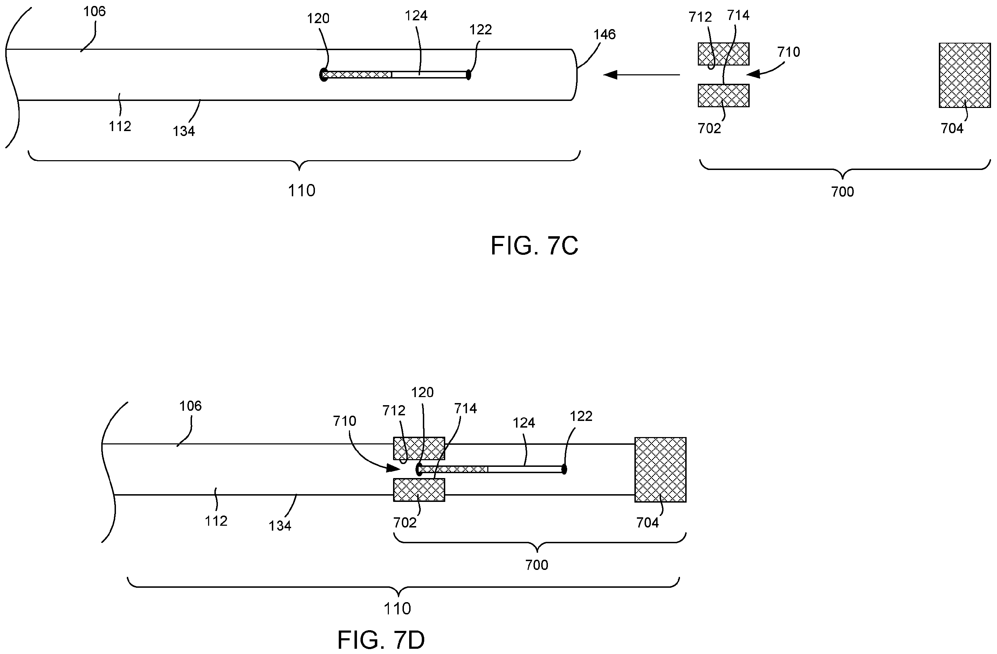

Referring particularly to Figs. 7C-7E, in a method of manufacturing the bipolar sphincterotome 102 and/or coupling the return electrode 700 to the elongate tubular member 106, the return electrode 700, including the proximal and distal cannulas 702, 704 may be proximally moved or inserted over the outer surface 134 of the elongate tubular member 106 from the distal end 146 of the elongate tubular member 106. In this context, the proximal movement is relative movement that is relative to the elongate tubular member 106. Such relative movement includes implementations where the proximal and distal cannulas 702, 704 are moved relative to the surrounding environment where the bipolar sphincterotome 102 is being assembled, and also includes implementations where the elongate tubular member 106 is moved relative to the surrounding environment (e.g., where the elongate tubular member 106, starting from the distal end 146, is inserted through the proximal and distal cannulas 702, 704) in order for the proximal and distal cannulas 702, 704 to be positioned or disposed about the outer surface 134 of the elongate tubular member 106 at desired or predetermined locations.

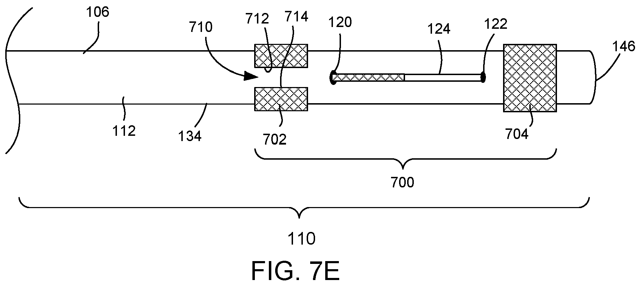

As previously described, in some implementations as in Figs. 7A-7E, the proximal cannula 702 may be circumferentially discontinuous, which may facilitate its relative proximal movement from the distal end 146 (Fig. 7C) to its final position proximal the opening 120 (Fig. 7E). For example, as shown in Figs. 7C-7E, a gap, slit, or other spacing 710 may circumferentially separate circumferential ends 712, 714 of the proximal cannula 702. As the proximal cannula 702 is proximally moved over the elongate tubular member 106 to its final position proximal the opening 120, the circumferential ends 712, 714 may proximally move past the anchor point 122 and then the opening 120, and/or the cutting edge 124 may move through the gap 710, as illustrated in Fig. 7D. This way, the cutting edge 124 does not interfere with the movement of the proximal cannula 702 as it proximally moves between the anchor point 122 and the opening 120.

In some of these implementations where the proximal cannula 702 is circumferentially discontinuous, a circumferential length of the proximal cannula 702 is at least 75% of the circumference of the elongate tubular member 106 about its outer surface 134, such that a circumferential length of the gap 710 is no more than 25% of the circumference of the elongate tubular member 106. Other implementations where the circumferential length of the proximal cannula 702 is less than 75% are possible.

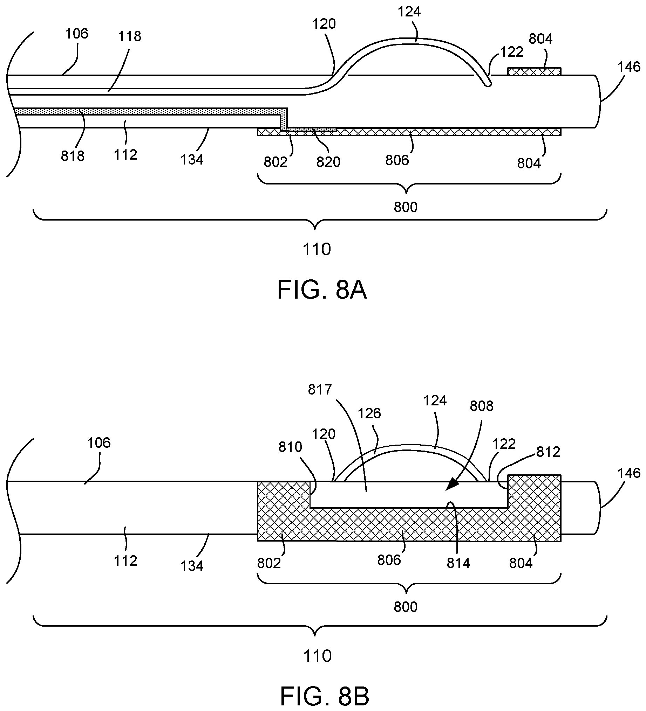

Figs. 8A-8E show an example implementation of the distal portion 110 of the bipolar sphincterotome 102 with a return electrode 800, a configuration of which may be representative of a fourth example configuration of the return electrode 132 of Figs. 1 and 2. The return electrode 800 may be similar to the configuration of the return electrode 700 in that it includes a proximal cannula 802 and a distal cannula 804. Additionally, similar to the implementation in Figs. 7A-7E, the proximal cannula 802 is circumferentially discontinuous and the distal cannula 804 is circumferentially continuous. In other implementations, the proximal and distal cannulas 802, 804 are both circumferentially discontinuous or are both circumferentially continuous. In addition or alternatively, in any of various implementations where the proximal and distal cannulas 802, 804 are both circumferentially discontinuous, the proximal and distal cannulas may have circumferential lengths that are the same as or different from each other.

In addition, unlike the configuration of the return electrode 700, the return electrode 800 may include a conductive coupling portion 806 that is longitudinally disposed between, physically attached to, and electrically connected to, both the proximal cannula 802 and the distal cannula 804. In some implementations, such as shown in Fig. 8B, the conductive coupling portion 806 may circumferentially extend about halfway around the outer surface 134 of the elongate tubular member 106. In any of various other implementations, the conductive coupling portion 806 may circumferentially extend more than halfway or less than halfway around the outer surface 134. In addition or alternatively, in some implementations as shown in Fig. 8B, over a longitudinal length of the conductive coupling portion 806, the circumferential length of the conductive coupling portion 806 is the same. However, in any of various other implementations, the circumferential length may be different over any two axial cross-sections over the longitudinal length of the conductive coupling portion 806.

Additionally, in some implementations, the proximal cannula 802, the distal cannula 804, and the conductive coupling portion 806 are integral components of a same, single, and/or continuous structure. For example, the return electrode 800 may be made of a single metallic cylindrical structure (e.g., sheet metal), and further, may be laser cut or otherwise have certain portions removed in order to form the proximal cannula 802, the distal cannula 804, and the conductive coupling portion 806 as a single or unitary structure made of the same material. In other implementations, the proximal cannula 802, the distal cannula 804, and the conductive coupling portion 806 may begin or formed as separate components that are connected together through an attachment process, such as soldering or welding, or using an epoxy or other adhesive material, as non-limiting examples. In addition or alternatively, the individual components, when respectively coupled or affixed to the elongate tubular member 106, may be disposed relative to each other such that they overlap or are otherwise physically connected to each other.

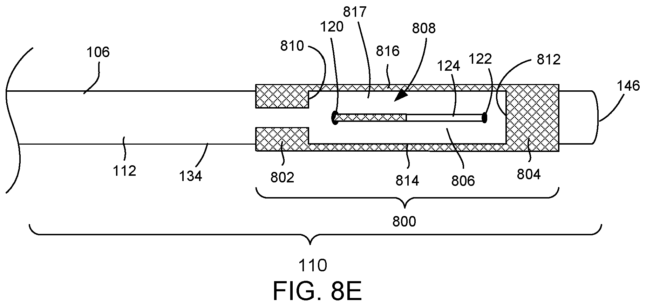

In addition, as shown in Figs. 8B-8E, the proximal cannula 802, the distal cannula 804, and the conductive coupling portion 806 may define a window 808. In particular, a distal end 810 of the proximal cannula 802, a proximal end 812 of the distal cannula 804, and opposing circumferential ends 814, 816 of the conductive coupling portion 806 may define a border of the window 808. As best shown in Fig. 8E, when the return electrode 800 is coupled to the elongate tubular member 106 in its desired or intended position, the border may surround an area or portion 817 of the outer surface 134 that includes both the opening 120 and the anchor point 122.

Additionally, as shown in Fig. 8A, a return wire 818, which may be representative of an example configuration of the return wire 136 of Figs. 1-3, may be configured similarly as the return wires 510, 606, 706 of the previously described implementations, in that the return wire 818 may longitudinally extend from within to outside of the elongate tubular member 106 at the distal portion 110 to electrically connect to the return electrode 800. Additionally, similar to the return wires 510, 606, 706, the return wire 818 may include a distal portion 820 disposed outside of the elongate tubular member 106 that is physically attached to the return electrode 800 in order to electrically connect the return wire 818 with the return electrode 800. In some of these implementations such as shown in Fig. 8A and similar to the configurations shown in Figs. 5A, 6A, 7A, the distal portion 820 is disposed between the outer surface 134 of the elongate tubular member 106 and the return electrode 800. In other implementations however, the distal portion 820 may extend over an outer surface of the return electrode 800, such that the return electrode 800 is disposed between the outer surface 134 of the return electrode 800. Additionally, similar to the configurations shown in Figs. 5A, 6A, and 7A, the distal portion 820 in Fig. 8A directly attached to the return electrode 800. In any of various other implementations, one or more of any of various types of conductive elements (e.g., solder, conductive ink, tape, etc.) may be used to electrically coupled the return electrode 800 and the return wire 818.

Additionally, in any of various implementations, the distal portion 820 may physically attach to at least one of the proximal cannula 802, the distal cannula 804, and the conductive coupling portion 806. Since the proximal cannula 802, the distal cannula 804, and the conductive coupling portion 806, in isolation, are physically and electrically connected to each other (e.g., by being integral components of the same metallic structure), then physically attaching the distal portion 820 to only one of the proximal cannula 802, the distal cannula 804, and the conductive coupling portion 806 electrically couples all of the proximal cannula 802, the distal cannula 804, and the conductive coupling portion 806 to the return wire 818. In the implementation in Fig. 8A, the distal portion 820 physically attaches to the proximal cannula 802. Other implementations where the distal portion 820 physically attaches to the distal cannula 804 and/or the conductive coupling portion 806 are possible.

In addition or alternatively, the return electrode 800 may be coupled and/or affixed to the outer surface 134 of the elongate tubular member 106 in any of various ways, such as by being glued or bonded (e.g., using an epoxy or other adhesive), crimped, and/or press-fit to and/or about the outer surface 134 of the elongate tubular member 106, as non-limiting examples. In addition or alternatively, in some implementations, the return electrode 800 may be fixedly attached to the body 112 of the elongate tubular member 106 by having at least a portion that grasps or grips the body 112, such as by being embedded in and/or by penetrating into the body 112. For example, proximal and/or distal ends of the return electrode 800 may be folded or curled into the body 112 to form a grasping or gripping engagement with the body 112. In addition or alternatively, the return electrode 800 may include one or more spikes, pins, or other similar sharp or piercing structure that can penetrate and grip onto the body 112 in order for the return electrode 800 to be affixed, and/or to enhance a coupling or attachment that the return electrode 800 has, to the body 112 of the elongate tubular member 106.

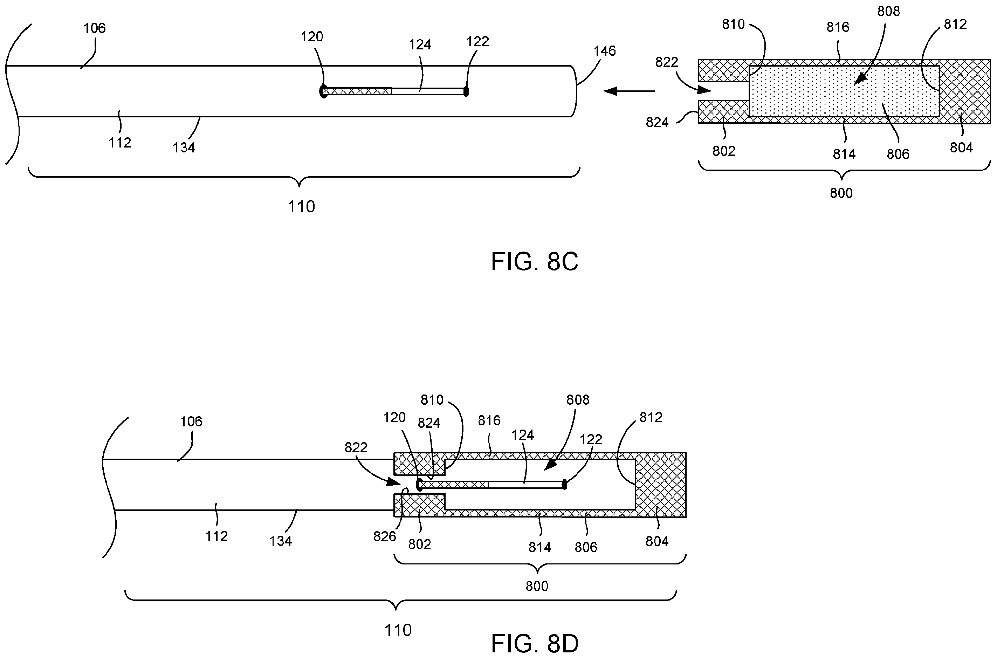

Additionally, in some implementations, the return electrode 800 may be coupled to the elongate tubular member 106 by being inserted over the elongate tubular member 106 from the distal end 146 of the elongate tubular member 106, similar to proximal and distal conductive cannulas 702, 704 as previously described with reference to Figs. 7C-7E. To illustrate, referring particularly to Figs. 8C-8E, in an example method of manufacturing the bipolar sphincterotome 102 and/or coupling the return electrode 800 to the elongate tubular member 106, the return electrode 800 may be proximally moved or inserted over the outer surface 134 of the elongate tubular member 106 from the distal end 146 of the elongate tubular member 106. In this context, the proximal movement is relative movement that is relative to the elongate tubular member 106. Such relative movement includes implementations where the return electrode 800 is moved relative to the surrounding environment where the bipolar sphincterotome 102 is being assembled, and also includes implementations where the elongate tubular member 106 is moved relative to the surrounding environment (e.g., where the elongate tubular member 106, starting from the distal end 146, is inserted through the return electrode 800) in order for the return electrode 800 to be positioned or disposed about the outer surface 134 of the elongate tubular member 106 at a desired or predetermined location relative to the opening 120, the anchor point 122, and/or the cutting edge 124.

Additionally, in some implementations as in Figs. 8A-8E, the proximal cannula 802 of the return electrode 800 may be circumferentially discontinuous, similar to the configuration of the proximal cannula 702. As with the proximal cannula 702, configuring the proximal cannula 802 to be circumferentially discontinuous may facilitate its relative proximal movement from the distal end 146 (Fig. 8C) to its final position proximal the opening 120 (Fig. 8E). For example, as shown in Figs. 8C-8E, a gap, slit, or other spacing 822 may circumferentially separate circumferential ends 824, 826 of the proximal cannula 802. As the proximal cannula 802 is proximally moved over the elongate tubular member 106 to its final position proximal the opening 120, the circumferential ends 810, 824 may proximally move past the anchor point 122 and then the opening 120, and/or the cutting edge 124 may move through the gap 822, as illustrated in Fig. 8D. This way, the cutting edge 124 does not interfere with the movement of the proximal cannula 802 as it proximally moves between the anchor point 122 and the opening 120.

In some of these implementations where the proximal cannula 802 is circumferentially discontinuous, a circumferential length of the proximal cannula 802 is at least 75% of the circumference of the elongate tubular member 106 about its outer surface 134, such that a circumferential length of the gap 822 is no more than 25% of the circumference of the elongate tubular member 106. Other implementations where the circumferential length of the proximal cannula 802 is less than 75% are possible.

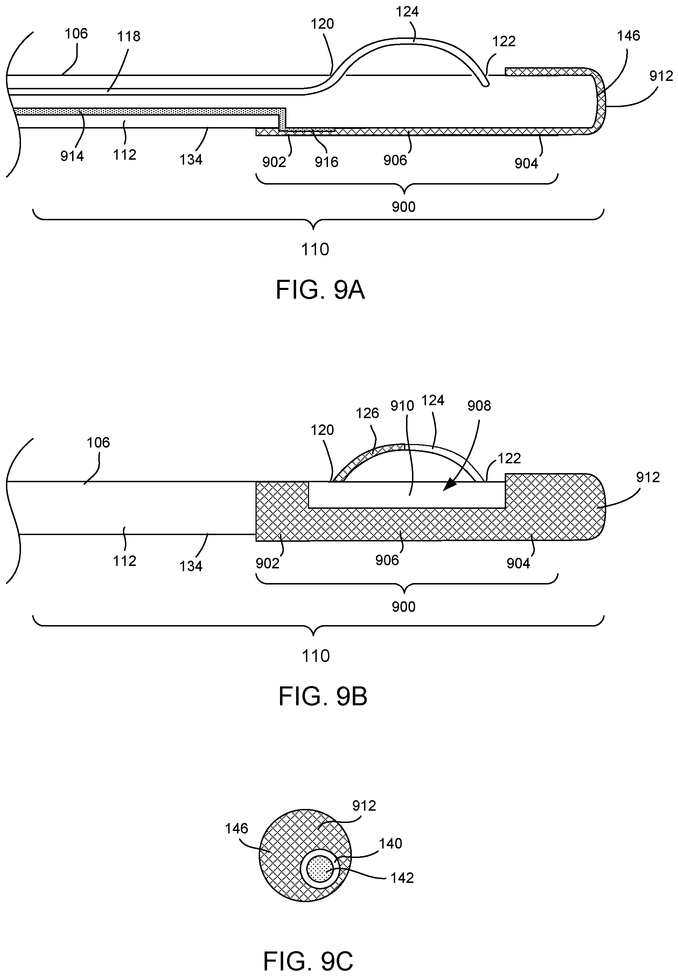

Figs. 9A-9C show an example implementation of the distal portion 110 of the bipolar sphincterotome 102 with a return electrode 900, a configuration of which may be representative of a fifth example configuration of the return electrode 132 of Figs. 1 and 2. The return electrode 900 may be similar to the configuration of the return electrode 800 in that it includes a proximal cannula 902, a distal cannula 904, and a conductive coupling portion 906 disposed between and physically and electrically connected to the proximal and distal cannulas 902, 904. In addition, the proximal cannula 902, the distal cannula 904, and the conductive coupling portion 906 may define a window 908, a border of which surrounds an area or portion 910 of the outer surface 134 that includes both the opening 120 and the anchor point 122.

The return electrode 900 may further include a distal tip (also called a distal end covering) 912 that covers the distal end 146 of the elongate tubular member 106. For at least some implementations such as shown in Figs. 9A, 9B, the distal tip 912 is a generally round and/or dome-shaped structure. The round and/or dome-shaped structure may provide an atraumatic structure in event that the distal tip 912 contacts tissue of a patient during delivery of the distal portion 110 to a treatment site within the patient and/or during performance of a sphincterotomy within the patient.

Additionally, the distal tip 912 is generally disposed distal the distal cannula 904. For at least some implementations such as shown in Figs. 9A, 9B, the distal tip 912 is physically attached to and/or distally projects from the distal cannula 904. Additionally for at least some implementations such as shown in Figs. 9A-9C, the distal tip 912 may completely cover a portion of the outer surface 134 of the elongate tubular member 106 distal the distal cannula 904, except the one or more lumens that extend all the way to the distal end 146. For example, as shown in Fig. 9C, the distal tip 912 covers the distal end 146 except for the wireguide lumen 140. Other implementations where the distal tip 912 does not cover other portions of the outer surface 134 besides the one or more lumens (e.g., the wireguide lumen 140) at the distal end 146 are possible. As a non-limiting example, the distal tip 912 may not cover a certain portion of the outer surface 134 surrounding the wireguide lumen 140 that sufficiently distances the distal tip 912 from the wireguide 142 so as to prevent arcing between the distal tip 912 and the wireguide 142.

Additionally, similar to the return electrode 800, in some implementations, the proximal cannula 902, the distal cannula 904, the conductive coupling portion 906, and the distal tip 912 may be components of a same or integral structure, such as the same piece of metal. For example, an elongate tubular structure with a dome-shaped tip may be formed from single piece of metal, and laser cutting or another metal removal process may be performed to create the window 908 and/or the spacing (not shown in Figs. 9A-9C) longitudinally extending through the proximal cannula 902. In other implementations, one or more of the proximal cannula 902, the distal cannula 904, the conductive coupling portion 906, and the distal tip 912 may be formed separate from the other components, and may be attached to the other components via an attachment process, such as soldering or welding or through application of an epoxy or other adhesive.

Additionally, the return electrode 900 may be coupled to the elongate tubular member 106 in the same or similar way as any of the return electrodes 500, 600, 700, 800, as previously described. For example, the return electrode 900 may be inserted over the elongate tubular member 106 from the distal end 146, such as previously described for the return electrodes 700 and 800 with reference to Figs. 7C-7E and 8C-8E, respectively. In addition or alternatively, the return electrode 900 may be coupled and/or affixed to the outer surface 134 of the elongate tubular member 106 in any of various ways, such as by being glued or bonded (e.g., using an epoxy or other adhesive), crimped, and/or press-fit to and/or about the outer surface 134 of the elongate tubular member 106, as non-limiting examples. In addition or alternatively, in some implementations, the return electrode 900 may be fixedly attached to the body 112 of the elongate tubular member 106 by having at least a portion that grasps or grips the body 112, such as by being embedded in and/or by penetrating into the body 112, such as previously described for any of the return electrodes 500, 600, 700, and 800.

Additionally, as shown in Fig. 9A, a return wire 914, which may be representative of an example configuration of the return wire 136 of Figs. 1-3, may be configured similarly as the return wires 510, 606, 706, 818 of the previously described implementations, in that the return wire 914 may longitudinally extend from within to outside of the elongate tubular member 106 at the distal portion 110 to electrically connect to the return electrode 900. The return wire 914 may include a distal portion 916 disposed outside of the elongate tubular member 106. Similar to the return wires 510, 606, 706, 818, in some implementations such as shown Fig. 9A, the distal portion 916 is disposed between the outer surface 134 of the elongate tubular member 106 and the return electrode 900. In other implementations, the distal portion 916 is disposed over the return electrode 900, such that the return electrode 900 is disposed between the outer surface 134 and the distal portion 916. Various ways of electrically connecting the return electrode 900 to the return wire 914 are possible.

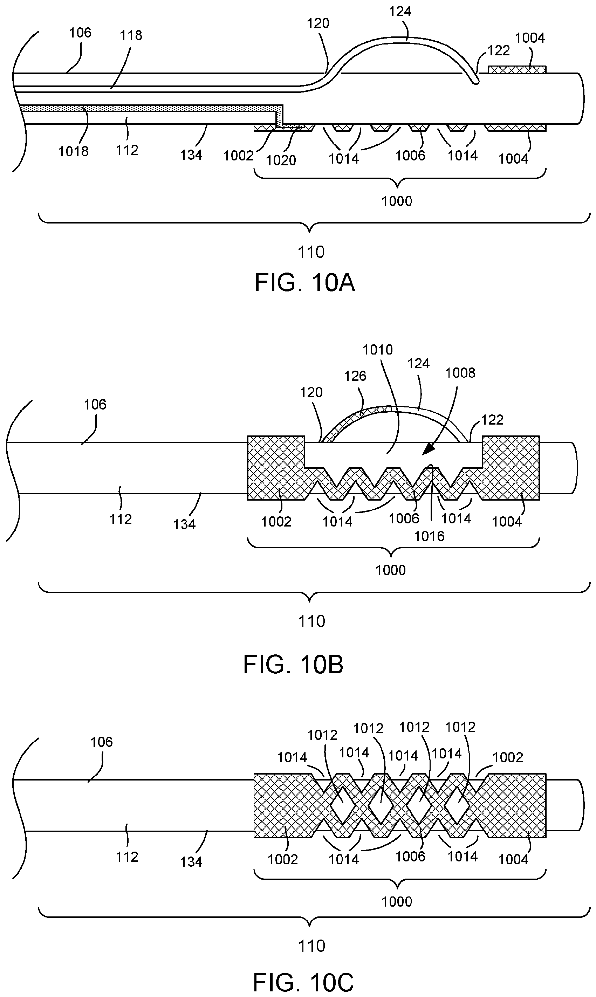

Figs. 10A-10C show an example implementation of the distal portion 110 of the bipolar sphincterotome 102 with a return electrode 1000, a configuration of which may be representative of a sixth example configuration of the return electrode 132 of Figs. 1 and 2. The return electrode 1000 may be similar to the configuration of the return electrode 800 in that it includes a proximal cannula 1002, a distal cannula 1004, and a conductive coupling portion 1006 disposed between and physically and electrically connected to the proximal and distal cannulas 1002, 1004. In addition, the proximal cannula 1002, the distal cannula 1004, and the conductive coupling portion 1006 may define a window 1008, a border of which surrounds an area or portion 1010 of the outer surface 134 that includes both the opening 120 and the anchor point 122.

The conductive coupling portion 1006 of the return electrode 1000 may further include or define at least one hole 1012 and/or at least one notch 1014 disposed on at least one of the circumferential ends or edges 1016 of the conductive coupling portion 1006. The at least one hole 1012 and/or the at least one notch 1014 may provide the conductive coupling portion 1016 with less material and/or surface area compared to other similar conductive coupling portions that do not have or define at least one hole and/or at least one notch (e.g., the conductive coupling portion 806, 906).

As previously described, in operation, the distal portion 110 of the bipolar sphincterotome 102 moves between curled and uncurled positions. Correspondingly, implementation of a metallic return electrode for the bipolar sphincterotome 102 may be enhanced by reducing the amount of metal longitudinally between the opening 120 and the anchor point 122 since that reduces the amount of metal that is bending or flexing during movement of the distal portion 110 between the curled and uncurled positions. This may be especially the case for the distal portion 110 at and/or around an apex of the distal portion 110 where the elongate tubular member 106 bends or flexes the most during movement between the curled and uncurled positions. In the example configuration of the return electrode 1000, the at least one hole 1012 and/or the at least one notch 1014 may serve to reduce the amount of material (e.g., metallic material) longitudinally between the opening 120 and the anchor point 122, in turn facilitating and/or enhancing the use of a metallic return electrode with a bipolar sphincterotome 102.

In some implementations such as best shown in Fig. 10C, the at least one hole 1012 includes a plurality of holes 1012. In particular of these implementations as in Fig. 10C, the plurality of holes 1012 longitudinally extend relative to each other, such as in a straight line parallel with the longitudinal axis of the elongate tubular member 106. Other implementations where at least some of the plurality of holes 1012 do not longitudinally extend and/or do not longitudinally extend in a straight line are possible. In addition or alternatively, in any of various implementations, each of the at least one hole 1012 has a predetermined shape, such as a polygonal shape. For example, in the example configuration in Fig. 10C, each of the holes 1012 has a diamond shape. Other implementations where the holes 1012 are shaped other than diamonds and/or where any two holes 1012 have different shapes are possible.

In addition, in some implementations, the at least one notch 1014 includes a plurality of notches 1014. In some of these implementations, the plurality of notches 1014 are disposed on both circumferential ends 1016 of the conductive coupling portion 1016, although other implementations where the plurality of notches are disposed on only one of the circumferential ends 1016 are possible.

Additionally, in some implementations such as in Figs. 10A-10C, at least a part of the conductive coupling portion 1006 includes or is in the form of at least one shape, such as a predetermined shape, as defined by the plurality of notches 1014. In some implementations such as in Figs. 10A-10C, the part in the form of at least one shape includes a plurality of sub-parts, where each of the sub-parts is in the form of a shape, as defined by the plurality of notches 1014. Any two of the sub-parts may have the same shape as, or different shapes from, each other in any of various implementations. For example, in the implementation shown in Fig. 10C, each sub-part is in the form of, or shaped as, a hexagon, as defined by the plurality of notches 1014. Correspondingly, as shown in Fig. 10C, each of the notches 1014 is triangular, half-diamond shaped or v-shaped, in turn defining a series of longitudinally extending hexagon-shaped sub-parts. Also, for at least some of these implementations such as shown in Fig. 10C, each of the least one hole 1012 is disposed within each of the at least one sub-part. For example, the configuration in Fig. 10C includes four holes 1012, each within a respective one of four hexagons of the conductive coupling portion 1006 defined by the notches 1014. Other implementations where none or at least one of the shapes of the conductive coupling portion 1006 defined by the notches 1014 do not have a hole disposed therein are possible.

Additionally, as shown in Fig. 10A, a return wire 1018, which may be representative of an example configuration of the return wire 136 of Figs. 1-3, may be configured similarly as the return wires 510, 606, 706, 818, 914 of the previously described implementations, in that the return wire 1018 may longitudinally extend from within to outside of the elongate tubular member 106 at the distal portion 110 to electrically connect to the return electrode 900. The return wire 914 may include a distal portion 1020 disposed outside of the elongate tubular member 106. Similar to the return wires 510, 606, 706, 818, 914, in some implementations such as shown Fig. 10A, the distal portion 1020 is disposed between the outer surface 134 of the elongate tubular member 106 and the return electrode 1000. In other implementations, the distal portion 1020 is disposed over the return electrode 1000, such that the return electrode 1000 is disposed between the outer surface 134 and the distal portion 1020. Various ways of electrically connecting the return electrode 1000 to the return wire 1018 are possible.

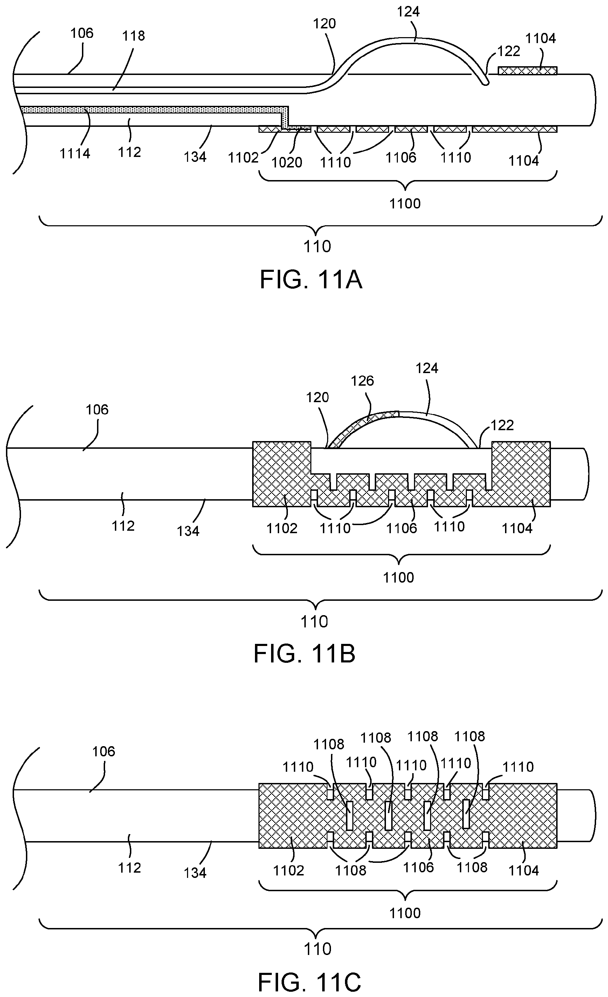

Figs. 11A-11C show an example implementation of the distal portion 110 of the bipolar sphincterotome 102 with a return electrode 1100, a configuration of which may be representative of a seventh example configuration of the return electrode 132 of Figs. 1 and 2. The return electrode 1100 is similar to the return electrode 1000 of Figs. 10A-10C, in that it includes a proximal cannula 1102, a distal cannula 1104, and a conductive coupling portion 1106 disposed between and connected to the proximal and distal cannulas 1102, 1104, and further includes at least one hole 1108 disposed in the conductive coupling portion 1106 and at least one notch 1110 disposed on at least one of the circumferential ends 1112 of the conductive coupling portion 1106.

However, instead of having diamond-shaped holes and triangular shaped notches as with the return electrode 1000, the at least one hole 1108 and the at least one notch 1110 are each rectangular. For at least some implementations, the rectangular notches 1110 may each be in the form of a slit or relatively thin gap or line having a circumferential length that is longer (e.g., at least two times longer) than its longitudinal width. Additionally, similar to the implementation of the return electrode 1000, at least a part of the return electrode 1100 includes or is in the form of at least one shape, as defined by the notches 1110. For example, as shown in Fig. 11C, the conductive coupling portion 1106 include a plurality of rectangles defined by the rectangular notches 1110. Similar to the implementation of the return electrode 1000, shapes other than rectangles may be used for any of various other implementations for the return electrode 1100.

In addition, as shown in Fig. 11A, a return wire 1114, which may be representative of an example configuration of the return wire 136 of Figs. 1-3, may be configured similarly as the return wires 510, 606, 706, 818, 914, 1018 of the previously described implementations, in that the return wire 1114 may longitudinally extend from within to outside of the elongate tubular member 106 at the distal portion 110 to electrically connect to the return electrode 1100. The return wire 914 may include a distal portion 1116 disposed outside of the elongate tubular member 106, that may further be disposed between the outer surface 134 of the elongate tubular member 106 and the return electrode 1100 as shown in Fig. 11A, or may be disposed over the return electrode 1100 in other implementations as previously described. Various ways of electrically connecting the return electrode 1100 to the return wire 1114 are possible.

Referring to both the return electrode 1000 of Figs. 10A-10C and the return electrode 1100 of Figs. 11A-11C, the holes 1012, 1108 and/or the notches 1014, 1110 may be formed in the respective return electrodes 1000, 1100 in any of various ways. As an example, the 1012, 1108 and the notches 1014, 1110 may be formed via laser cutting, although other ways of forming the holes 1012, 1108 and/or the notches 1014, 1110 are possible.

Additionally, similar to the return electrodes 700, 800, 900, in some implementations, the return electrodes 1000, 1100 may be coupled to the elongate tubular member 106 by being inserted over the distal end 146. In addition or alternatively, the return electrodes 1000, 1100 may be coupled and/or affixed to the outer surface 134 of the elongate tubular member 106 in any of various ways, such as by being glued or bonded (e.g., using an epoxy or other adhesive), crimped, and/or press-fit to and/or about the outer surface 134 of the elongate tubular member 106, as non-limiting examples.

Additionally, the configurations of the return electrode 1000 of Figs. 10A-10C and the return electrode 1100 of Figs. 11A-11C are merely examples. Other configurations for the return electrode 1000 and/or the return electrode 1100, where the conductive coupling portion 1006, 1106 includes at least one hole and/or at least one notch, in any of various other implementations are possible. For example, the shapes of the holes 1012, 1108 may be different than diamond shaped and rectangular, such as circular, oval-shaped or any of various types of polygonal shapes, such as triangular, trapezoidal, pentagonal, hexagonal, or octagonal, as non-limiting examples. In addition or alternatively, the shapes of the notches 1014, 1110 may be different than triangular and rectangular, such as semi-circular, semi-oval shaped, or trapezoidal, as non-limiting examples. In addition or alternatively, for either of the return electrode 1000 or the return electrode 1100, any two holes 1012, 1108 and/or any two notches 1014, 1110 may have the same shape as, or different shapes from, each other. In addition or alternatively, the sizes of the holes 1012, 1108 and/or the notches 1014, 1110 shown in Figs. 10A-10C and 11A-11C relative to the elongate tubular member 106 are merely exemplary and may be smaller or larger and/or their respective dimensions may be proportionately different from the example configurations shown in Figs. 10A-10C and 11A-11C. In addition or alternatively, the shapes of the conductive coupling portions 1006, 1106 defined the notches 1014, 1110 may be different from hexagons and rectangles in any of various other implementations, such as circular, oval-shaped, diamond-shaped, or octagonal, as non-limiting examples. In addition or alternatively, the shapes may in the form of any of various patterns. For example, in the return electrode 1000 in Figs. 10A-10C, the holes 1012 and notches 1014 and the hexagons they define may be considered to form a cross-hatch pattern, although other cross-hatch patterns may have different configurations in any of various other implementations. In addition or alternatively, some other implementations may include at least one hole 1012, 1108 and no (i.e., zero) notches 1014, 1110. Still other implementations may include at least one notch 1014, 1110 and no (i.e., zero) holes 1012, 1108. In addition or alternatively, in some other implementations, the at least one hole 1012, 1108 includes only one hole, which may have any of various sizes, dimensions, and shapes. In some of these implementations, the only one hole may longitudinally extend more than half a total longitudinal length of the conductive coupling portion 1006, 1106 and/or may circumferentially extend more than half a total circumferential length of the conductive coupling portion 1006, 1106. Various ways of implementing at least one hole and/or at least one notch in a conductive coupling portion disposed between and connected to a proximal cannula disposed proximal the opening 120 and a distal cannula disposed distal the anchor point 122 are possible.

Various other implementations of a return electrode that incorporate one of more of the features from the return electrodes 500, 600, 700, 800, 900, 1000, 1100 or combinations thereof are possible. For example, other implementations of a return electrode may combine features from the return electrodes 600 and 700, such as by having the proximal semi-ring 602 and the distal cannula 704, or the proximal cannula 702 and the distal semi-ring 604. As other examples, other implementations of a return electrode may include only the proximal semi-ring 602 and not the distal semi-ring 604; only the distal semi-ring 604 and not the proximal semi-ring 602; only the proximal cannula 702/802/902/1002/1102 and not the distal cannula 704/804/904/1004/1104; or only the distal cannula 704/804/904/1004/1104 and not the proximal cannula 702/802/902/1002/1102. As another example, other implementations of a return electrode may incorporate the distal tip 912 from the return electrode 900 of Figs. 9A-9C into any of the return electrodes 700, 800, 1000, 1100, such as by connecting the distal tip 912 to any of the distal cannulas 704, 804, 1004, 1104. As still another example, other implementations of a return electrode may incorporate the at least one hole 1012 or 1108 and/or the least one notch 1014 or 1110 into any of the return electrodes 800 or 900, such as by integrating the at least one hole 1012 or 1108 and/or the least one notch 1014 or 1110 with the conductive coupling portion 806 or 906. Various other implementations of a return electrode that combine the features of the return electrodes 500, 600, 700, 800, 900, 1000, 1100 are possible.

The subject matter of the present description may also relate, among others, to the following aspects:
In a first aspect, includes a bipolar sphincterotome that includes: an elongate tubular member longitudinally extending from a proximal portion to a distal portion; a cutting wire longitudinally extending in the elongate tubular member, and extending to outside of the elongate tubular member via a cutting wire opening in the elongate tubular member, wherein a distal end of the cutting wire is anchored to the elongate tubular member at an anchor point distal the cutting wire opening, and wherein a portion of the cutting wire outside of the elongate tubular member comprises a cutting edge; a return wire longitudinally extending from the proximal portion to the distal portion; and a return electrode electrically coupled to the return wire, the return electrode comprising a proximal cannula disposed proximal the cutting wire opening and a distal cannula disposed distal the anchor point, both the proximal cannula and the distal cannula electrically coupled to the return wire, and wherein the proximal cannula and the distal cannula are both metallic.

A second aspect includes the first aspect, and further includes wherein the proximal cannula comprises a gap separating circumferential ends of the proximal cannula.

A third aspect includes any of the first or second aspects, and further wherein the return electrode further includes: a conductive coupling portion longitudinally disposed between and connected to the proximal cannula and the distal cannula.

A fourth aspect includes the third aspect, and further includes wherein the proximal cannula, the distal cannula, and the conductive coupling portion define a window having a border that surrounds the cutting wire opening and the anchor point.

A fifth aspect includes any of the third or fourth aspects, and further includes wherein the proximal cannula, the distal cannula, and the conductive coupling portion are integral components of a same piece of metallic material.

A sixth aspect includes any of the first through fifth aspects, and further includes wherein the return electrode further includes a conductive dome tip attached to the distal cannula and covering a distal end of the elongate tubular member.

A seventh aspect includes the sixth aspect, and further includes wherein the conductive dome tip does not cover an opening of a wireguide lumen at the distal end of the elongate tubular member.

An eighth aspect includes any of the third through seventh aspects, and further includes wherein the conductive coupling portion includes at least one hole circumferentially disposed between a first circumferential edge and a second circumferential edge of the conductive coupling portion.

A ninth aspect includes the eighth aspect, and further includes wherein the at least one hole comprises a plurality of holes longitudinally extending in the conductive coupling portion.

A tenth aspect includes any of the third through ninth aspects, and further includes wherein the conductive coupling portion includes at least one notch on at least one of a first circumferential edge of the conductive coupling portion or a second circumferential edge of the conductive coupling portion.

An eleventh aspect includes the tenth aspect, and further includes wherein the at least one notch comprises at least one first notch on the first circumferential edge and at least one second notch on the second circumferential edge.

A twelfth aspect includes any of the tenth or eleventh aspects, and further includes wherein a part of the conductive coupling portion has a shape defined by the at least one notch.

A thirteenth aspect includes the twelfth aspect, and further includes wherein the part includes a plurality of longitudinally extending sub-parts connected to each other, where each of the sub-parts has the shape.

A fourteenth aspect includes any of the twelfth or thirteenth aspects, and further includes wherein the shape includes a hexagon.

A fifteenth aspect includes any of the twelfth or thirteenth aspects, and further includes wherein the shape includes a rectangle.

A sixteenth aspect includes any of the tenth through fourteenth aspects, and further includes wherein the at least one notch includes at least one v-shaped notch.

A seventeenth aspect includes any of the tenth through thirteenth or fifteenth aspects, and further includes wherein the at least one notch includes a rectangular notch.

An eighteenth aspect includes a method of forming a return electrode on a distal portion of a bipolar sphincterotome, including: moving a proximal cannula to a first final position proximal a cutting wire opening of the bipolar sphincterotome and a distal cannula to a second final position distal an anchor point of the bipolar sphincterotome, wherein the proximal cannula and the distal cannula are both metallic; and electrically coupling a return wire of the bipolar sphincterotome to both the proximal cannula and the distal cannula.

A nineteenth aspect includes the eighteenth aspect, and further includes wherein moving the proximal conductive cannula and the distal conductive cannula comprises: proximally moving the proximal cannula and the distal cannula from a distal end of the elongate tubular member to the first final position and the second final position, respectively.

A twentieth aspect includes any of the eighteenth or nineteenth aspects, and further includes wherein the bipolar sphincterotome further includes a conductive coupling portion longitudinally disposed between and connected to the proximal cannula and the distal cannula, and wherein moving the proximal cannula and the distal cannula includes: moving the proximal cannula, the distal cannula, and the conductive coupling portion in unison over the elongate tubular member.

A twenty-first aspect includes any of the eighteenth through twentieth aspects, and further includes: exposing the cutting wire opening and the anchor point with a window having a border defined by the proximal cannula, the distal cannula, and the conductive coupling portion.

A twenty-second aspect includes any of the eighteenth through twenty-first aspects, and further includes: covering a distal end of the elongate tubular member with a conductive dome tip attached to the distal cannula.

A twenty-third aspect includes the twenty-second aspect, and further includes wherein the conductive dome tip covers the distal end of the elongate tubular member without covering an opening of a wireguide lumen at the distal end.

A twenty-fourth aspect includes any of the eighteenth through twenty-third aspects, and further includes wherein a gap longitudinally extends through the proximal cannula, and wherein moving the proximal cannula comprises moving a cutting edge of the cutting wire through the gap when the proximal cannula moves past the anchor point and the cutting wire opening.

The foregoing description of various embodiments of the invention has been presented for purposes of illustration and description. It is not intended to be exhaustive or to limit the invention to the precise embodiments disclosed. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

## Claims

1. A bipolar sphincterotome comprising:
an elongate tubular member longitudinally extending from a proximal portion to a distal portion;
a cutting wire longitudinally extending in the elongate tubular member, and extending to outside of the elongate tubular member via a cutting wire opening in the elongate tubular member, wherein a distal end of the cutting wire is anchored to the elongate tubular member at an anchor point distal the cutting wire opening, and wherein a portion of the cutting wire outside of the elongate tubular member comprises a cutting edge;
a return wire longitudinally extending from the proximal portion to the distal portion; and
a return electrode electrically coupled to the return wire, the return electrode comprising a proximal cannula disposed proximal the cutting wire opening and a distal cannula disposed distal the anchor point, both the proximal cannula and the distal cannula electrically coupled to the return wire, and
wherein the proximal cannula and the distal cannula are both metallic.

2. The bipolar sphincterotome of claim 1, wherein the proximal cannula comprises a gap separating circumferential ends of the proximal cannula.

3. The bipolar sphincterotome of any of claims 1 or 2, wherein the return electrode further comprises:
a conductive coupling portion longitudinally disposed between and connected to the proximal cannula and the distal cannula, for example wherein the proximal cannula, the distal cannula, and the conductive coupling portion define a window having a border that surrounds the cutting wire opening and the anchor point, and/or wherein the proximal cannula, the distal cannula, and the conductive coupling portion are integral components of a same piece of metallic material.

4. The bipolar sphincterotome of any of claims 1 to 3, wherein the return electrode further comprises a conductive dome tip attached to the distal cannula and covering a distal end of the elongate tubular member, for example wherein the conductive dome tip does not cover an opening of a wireguide lumen at the distal end of the elongate tubular member.

5. The bipolar sphincterotome of any of claims 3 to 4, wherein the conductive coupling portion comprises at least one hole circumferentially disposed between a first circumferential edge and a second circumferential edge of the conductive coupling portion, for example wherein the at least one hole comprises a plurality of holes longitudinally extending in the conductive coupling portion.

6. The bipolar sphincterotome of any of claims 3 to 5, wherein the conductive coupling portion comprises at least one notch on at least one of a first circumferential edge of the conductive coupling portion or a second circumferential edge of the conductive coupling portion, for example wherein the at least one notch comprises at least one first notch on the first circumferential edge and at least one second notch on the second circumferential edge.

7. The bipolar sphincterotome of claim 6, wherein a part of the conductive coupling portion has a shape defined by the at least one notch, for example wherein the part comprises a plurality of longitudinally extending sub-parts connected to each other, where each of the sub-parts has the shape.

8. The bipolar sphincterotome of claim 7, wherein the shape comprises one of: (a) a hexagon; and (b) a rectangle.

9. The bipolar sphincterotome of any of claims 6 to 8, wherein the at least one notch comprises at least one v-shaped notch.

10. The bipolar sphincterotome of claim 6, 7, or 8, wherein the at least one notch comprises a rectangular notch.

11. A method of forming a return electrode on a distal portion of a bipolar sphincterotome, the method comprising:
moving a proximal cannula to a first final position proximal a cutting wire opening of the bipolar sphincterotome and a distal cannula to a second final position distal an anchor point of the bipolar sphincterotome, wherein the proximal cannula and the distal cannula are both metallic; and
electrically coupling a return wire of the bipolar sphincterotome to both the proximal cannula and the distal cannula.

12. The method of claim 11, wherein moving the proximal cannula and the distal cannula comprises:
proximally moving the proximal cannula and the distal cannula from a distal end of the elongate tubular member to the first final position and the second final position, respectively.

13. The method of any of claims 11 or 12, wherein the bipolar sphincterotome further comprises a conductive coupling portion longitudinally disposed between and connected to the proximal cannula and the distal cannula, and wherein moving the proximal cannula and the distal cannula comprises:
moving the proximal cannula, the distal cannula, and the conductive coupling portion in unison over the elongate tubular member.

14. The method of any of claims 11 to 13, further comprising at least one of:
(a) exposing the cutting wire opening and the anchor point with a window having a border defined by the proximal cannula, the distal cannula, and the conductive coupling portion; and
(b) covering a distal end of the elongate tubular member with a conductive dome tip attached to the distal cannula, for example wherein the conductive dome tip covers the distal end of the elongate tubular member without covering an opening of a wireguide lumen at the distal end.

15. The method of any of claims 11 to 14, wherein a gap longitudinally extends through the proximal cannula, and wherein moving the proximal cannula comprises moving a cutting edge of the cutting wire through the gap when the proximal cannula moves past the anchor point and the cutting wire opening.
